# EUROPÄISCHE PATENTSCHRIFT

(11) **EP 1 143 923 B1**
(45) Veröffentlichungstag und Bekanntmachung des Hinweises auf die Patenterteilung: **06.05.2004**
(21) Anmeldenummer: 00971366.0
(22) Anmeldetag: 12.10.2000
(51) Int. Cl.: A61K 7/13, D06M 13/35

(54) **MITTEL UND VERFAHREN ZUR FÄRBUNG VON FASERN**
AGENT AND METHOD FOR COLOURING FIBRES
AGENT ET PROCEDE POUR LA COLORATION DE FIBRES

(30) Priorität: 20.10.1999 DE 19950404
(43) Veröffentlichungstag der Anmeldung: 17.10.2001
(73) Patentinhaber: Wella Aktiengesellschaft, 64274 Darmstadt (DE)
(72) Erfinder: JAVET, Manuela, CH-1723 Marly (CH); MUELLER, Catherine, CH-1723 Marly (CH)
(86) Internationale Anmeldenummer: PCT/EP2000/010049
(87) Internationale Veröffentlichungsnummer: WO 2001/028507

(56) Entgegenhaltungen:
- EP-A- 0 873 744
- EP-A- 0 873 745
- DE-A- 19 745 356

## Beschreibung

Gegenstand der vorliegenden Erfindung ist ein Mittel zur Färbung von Fasern, insbesondere Keratinfasern (z.B. menschlichen Haaren), das eine Kombination aus einem 1-Alkyl-methylchinolinium Salz der Formel (I) oder (II) und Carbonylverbindungen enthält, ein Verfahren zum Färben von Fasern, insbesondere Keratinfasern, sowie ein Mehrkomponenten-Kit zum Färben und späteren Entfärben von Fasern.

Haarfärbemittel werden je nach zu färbender Ausgangshaarfarbe und gewünschtem Endresultat hauptsächlich in den Bereich der Oxidationsfärbemittel oder der Tönungen unterteilt.

Oxidationshaarfarben eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden.

Direktziehende Farbstoffe sind hauptsächlich in nicht-oxidativen Färbemitteln (sogenannten Tönungsmittein) enthalten. Einige direktziehende Farbstoffe wie z. B. die Nitrofarbstoffe, können aufgrund ihrer geringen Größe in das Haar eindringen und es - zumindestens in den äußeren Bereichen - direkt anfärben. Derartige Tönungen sind sehr haarschonend und überstehen in der Regel 6 bis 8 Haarwäschen.

Direktziehende Farbstoffe, insbesondere Nitrofarbstoffe, werden ebenfalls häufig in oxidativen Färbemitteln zur Erzeugung bestimmter Nuancen beziehungsweise zur Intensivierung der Farbe eingesetzt.

Es ist bekannt, daß oxidativ im Haar erzeugte farbige Polymere im allgemeinen sehr haltbar gegen äußere Einflüsse wie Wasser, Shampoo oder Licht sind. Je nach Färbetechnik sind sie so fest verankert, daß sie bis zum nächsten Haarschnitt im Haar verbleiben. Die Verwendung von Wasserstoffperoxid - insbesondere im alkalischen Milieu - wirkt sich allerdings nachteilig auf die Haarstruktur aus.

Aus der DE-OS 197 45 292 ist die Verwendung einer Kombination von Malonaldehydderivaten, wie zum Beispiel Malonaldehyd-bisdialkylacetalen, und Aminen oder CH-aciden Verbindungen zur Färbung von Haaren ohne Zusatz von Oxidationsmitteln bekannt. Ebenfalls ist aus dem Stand der Technik die Verwendung einer Kombination von bestimmten Aldehyden (DE-OS 197 17 280: bestimmte heterozyklische Carbonylverbindungen; DE-OS 197 17 223: bestimmte Aminovinylaldehyde; DE-OS 197 17 224: bestimmte ungesättigte, nicht-aromatische Aldehyde; DE-OS 197 45 356: bestimmte Oniumaldehyde oder,Oniumketone) und Aminen oder CH-aciden Verbindungen zur Färbung von Haaren ohne Zusatz von Oxidationsmitteln bekannt. Diese Färbemittel erfüllen die gestellten Anforderungen jedoch nur teilweise.

Es besteht daher weiterhin ein großer Bedarf für Färbemittel, die unter milden Bedingungen sowohl intensive als auch schonende Färbungen mit einer breiten Nuancenpalette ermöglichen.

Überraschenderweise wurde nunmehr gefunden, daß diese Aufgabe in hervorragender Weise durch den Einsatz eines ein Alkanolamin, mindestens eine Carbonylverbindung, insbesondere eine aromatische (nicht-heterozyklische) Aldehydverbindung, und mindestens eine Verbindung der Formel (I) oder (II),

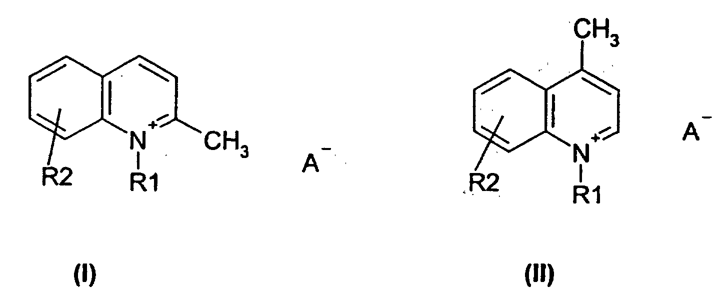

in der R1 für eine C1-C3-Alkylgruppe, C1-C3-Alkoxyalkylgruppe oder C1-C3-Hydroxyalkylgruppe steht und R2 gleich Wasserstoff, einer Hydroxygruppe, einer Methoxygruppe, einem Halogenatom , einer Aminogruppe oder einer Dimethylaminogruppe ist und A⁻ ein Anion, beispielsweise Chlorid, Bromid, Sulfat, Hydrogensulfat, Monomethylsulfat, Acetat, Lactat oder Jodid, darstellt; enthaltenden Mittels zur Färbung von Fasern, insbesondere Keratinfasern, wie zum Beispiel menschlichen Haaren, gelöst wird.

Die einzelnen Komponenten des erfindungsgemäßen Färbemittels werden bis kurz vor der Anwendung voneinander getrennt aufbewahrt und erst kurz vor der Anwendung zu einem gebrauchsfertigen Färbemittel vermischt und sodann auf die zu färbende Faser aufgetragen. Hierbei sind mehrere Varianten möglich. So können die Verbindungen der Formel (I) und/oder (II) gemeinsam mit der Aldehydverbindung sowie gegebenenfalls direktziehenden Farbstoffen (=Farbträgermasse) abgepackt werden und kurz vor der Anwendung mit dem Alkanolamin vermischt werden. Es ist jedoch ebenfalls möglich, das erfindungsgemäße Färbemittel in Form eines 2-Komponenten-Mittels zu konfektionieren, bestehend aus einer Farbträgermasse (a), welche die Verbindungen der Formel (I) und/oder (II) sowie das Monoalkanolamin und gegebenenfalls direktziehende Farbstoffe enthält, und einer weiteren Farbträgermasse (b), welche die Aldehydverbindung und gegebenenfalls direktziehende Farbstoffe enthält. Eine weitere Möglichkeit ist ein 2-Komponenten-Mittel bestehend aus einer Farbträgermasse (a), welche die Verbindungen der Formel (I) und/oder (II) und gegebenenfalls direktziehende Farbstoffe enthält, und einer weiteren Farbträgermasse (b), welche die Aldehydverbindung sowie das Monoalkanolamin und gegebenenfalls direktziehende Farbstoffe enthält. Selbstverständlich ist es ebenfalls möglich die 3 Komponenten in Form eines 3-Komponenten-Mittels zu konfektionieren, das aus einer Farbträgermasse (a), welche die Verbindungen der Formel (I) und/oder (II) und gegebenenfalls direktziehende Farbstoffe enthält, und einer weiteren Farbträgermasse (b), welche die Aldehydverbindung und gegebenenfalls direktziehende Farbstoffe enthält, und einer Komponente (c), welche das Alkanolamin enthält, besteht.

Unter den Verbindungen der Formeln (I) und (II) sind die folgenden Verbindungen besonders bevorzugt: 1-Ethyl-2-methyl-chinoliniumjodid, 1-Ethyl-2-methyl-chinoliniumchlorid, 1-Ethyl-4-methyl-chinoliniumjodid und 1-Ethyl-4-methyl-chinoliniumchlorid.

Die Verbindungen der Formeln (I) und (II) sind zum Teil im Handel erhältlich. Die Verbindungen der Formeln (I) und (II) können jedoch auch nach aus der Literatur bekannten üblichen Syntheseverfahren hergestellt werden. In diesem Zusammenhang sei insbesondere auf die Artikel von R. Long und K. Schofield in J. Chem. Soc., 3161 (1953),
E. Roberts und E. E. Turner in J. Chem. Soc., 1832 (1927),
E. A. Fehnel in J. Org. Chem., 31, 2899 (1966) und
C.-C. Cheng and S. -J. Yan, "The Friedländer synthesis of quinolines", in Org. React., 28, 37, (1982) hingewiesen, in denen die Herstellung der nicht-N-alkylierten Chinoline beschrieben wird. Ein Verfahren zur Alkylierung des Chinolinstickstoffs wird beispielsweise in Houben-Weyl, "Methoden der organischen Chemie, Hetarene II-Teil1", R. Kreher (Hrsg.), Georg Thieme-Verlag, Bd. E 7a, Seite 553 (1991), beschrieben.

Als Aldehydverbindung der Komponente (b) können verwendet werden: Vanillin (4-Hydroxy-3-methoxy-benzaldehyd), Isovanillin (3-Hydroxy-4-methoxy-benzaldehyd), 3,4-Dihydroxy-benzaldehyd, 4-Hydroxybenzaldehyd, 3,5-Dimethoxy-4-hydroxy-benzaldehyd, 4-Dimethylaminobenzaldehyd, 4- Dimethylamino-zimtaldehyd, 4-Hydroxy-2-methoxybenzaldehyd, 3,5-Dimethyl-4-hydroxybenzaldehyd, 4-Dimethylamino-2-methoxybenzaldehyd, 2-Hydroxybenzaldehyd, 4-Hydroxy-1-naphthaldehyd, 4-Methoxy-1-naphthatdehyd, 4-Dimethylamino-1-naphthaldehyd, 4'-Hydroxy-biphenyl-1-carbaldehyd, 2-Hydroxy-3-methoxybenzaldehyd, 2,4-Dihydroxy-benzaldehyd, 3,4-Dihydroxybenzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxy-benzaldehyd, 2,5-Dimethoxybenzaldehyd, 3,5-Dimethoxy-benzaldehyd, 3,4-Dimethoxybenzaldehyd, Benzol-1,4-dicarbaldehyd, 4-Ethoxy-benzaldehyd, 2-Methyl-1,4-naphthochinon, 4-Carboxybenzaldehyd, 4-Hydroxy-3-methoxyzimtaldehyd, 3,5-Dimethoxy-4-hydroxy-zimtaldehyd, 3-Methoxy-4-(1-pyrrolidinyl)-benzaldehyd, 4-Diethylamino-3-methoxy-benzaldehyd, 1,2-Phthaldialdehyd, 4-Dibutylamino-benzaldehyd, 4-Diethylamino-2-hydroxybenzaldehyd, 3,4-Dimethoxy-5-hydroxy-benzaldehyd, 5-(4-(Diethylamino)phenyl)-2,4-pentadienal, 2-Methoxy-1-naphthaldehyd, 3-Ethoxy-4-hydroxybenzaldehyd, 2-Nitrobenzaldehyd, 3-Nitrobenzaldehyd und 4-Nitrobenzaldehyd.

Als Alkanolamin kommen Monethanolamin, Isopropanolamin und Diisopropanotamin in Betracht, wobei Isopropanolamin und insbesondere Monethanolamin bevorzugt sind. Der Zusatz des Alkanolamins erfolgt in einer Menge, die ausreichend ist, um das erfindungsgemäße Mittel auf den gewünschten pH-Wert einzustellen.

Die Verbindungen der Formel (I) und/oder (II) und die Aldehyd-verbindungen sind in der jeweiligen Farbträgermasse jeweils in einer Gesamtmenge von etwa 0,02 bis 20 Gewichtsprozent, vorzugsweise 0,2 bis 10 Gewichtsprozent, enthalten, wobei in dem durch Vermischen der einzelnen Komponenten erhaltenen gebrauchsfertigen Färbemittel die Verbindung der Formel (I) und/oder (II) und die Aldehydverbindung jeweils in einer Gesamtmenge von etwa 0,01 bis 10 Gewichtsprozent, vorzugsweise 0,1 bis 5 Gewichtsprozent, enthalten ist.

Weiterhin kann das erfindungsgemäße Färbemittel gegebenenfalls zusätzlich übliche, physiologisch unbedenkliche, direktziehende Farbstoffe aus der Gruppe der Nitrofarbstoffe, Azofarbstoffe, Chinonfarbstoffe und Triphenylmethanfarbstoffe enthalten, beispielsweise 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl)amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-(2-Aminoethylamino)-4-[di(2-hydroxyethyl)amino]-2-nitrobenzol, 1-Amino-4-[(2-hydroxyethyl)-amino]-2-nitrobenzol (HC Red No. 7), 2-Amino-4,6-dinitro-phenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-((2-Hydroxyethyl)methylamino)-1-(methylamino)-2-nitrobenzol, 1-Amino-4-((2,3-dihydroxypropyl)amino)-5-methyl-2-nitrobenzol, 1-Amino-4-(methylamino)-2-nitrobenzol, 4-Amino-2-nitro-1-((prop-2-en-1-yl)amino)-benzol, 4-Amino-3-nitrophenol, 4-[(2-Hydroxyethyl)amino]-3-nitrophenol1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 6-Amino-3-((2-hydroxyethyl)-amino)-2-nitropyridin, 3-Amino-6-((2-hydroxyethyl)amino)-2-nitropyridin, 3-Amino-6-(ethylamino)-2-nitropyridin, 3-((2-Hydroxyethyl)amino)-6-(methylamino)-2-nitropyridin, 3-Amino-6-(methylamino)-2-nitropyridin, 6-(Ethylamino)-3-((2-hydroxyethyl)amino)-2-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxyethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-(Di(2-hydroxyethyl)amino)-5-nitrophenol, 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)-amino]-4-nitrobenzol, 4-[(2,3-Dihydroxypropyl)-amino]-3-nitro-1-trifluormethyl-benzol (HC Yellow No. 6), 1-Chlor-2,4-bis[(2-hydroxyethyl)amino]-5-nitrobenzol (HC Yellow No. 10), 1-Amino-4-((2-aminoethyl)-amino)-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 3-((2-Hydroxyethyl)amino)-4-methyl-1-nitrobenzol, 4-Chlor-3-((2-hydroxyethyl)amino)-1-nitrobenzol, 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 9-(Dimethylamino)-benzo[a]phenoxazin-7-ium-chlorid (CI51175; Basic Blue No. 6), Di[4-(diethylamino)phenyl][4-(ethylamino)naphthyl]carbenium-chlorid (CI42595; Basic Blue No. 7), Di-(4-(dimeihylamino)phenyl)-(4-(methylphenylamino)naphthalin-1-yl)carbenium-chlorid (CI42563; Basic Blue No. 8), 3,7-Di(dimethylamino)phenothiazin-5-ium-chlorid (CI52015; Basic Blue No. 9), Di[4-(dimethylamino)phenyl][4-(phenylamino)naphthyl]-carbeniumchlorid (CI44045; Basic Blue No. 26), 2-[(4-(Ethyl(2-hydroxyethyl)amino)phenyl)azo]-6-methoxy-3-methyl-benzothiazofiummethylsulfat (CI11154; Basic Blue No. 41 ), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)phenyl][4-(methylamino)phenyl]carbenium-chlorid (CI42535; Basic Violet No. 1), Tris[4-(dimethylamino)phenyl]carbenium-chlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010:Basic Brown No. 4), 1-[(4-Aminophenyl)azo]-7-(trimethylammonio)-2-naphthol-chlorid (CI12250; Basic Brown No. 16), 1-[(4-Amino-2-nitrophenyl)azo)-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)-phenyl)azo]-pyrazol-5-on-chlorid (CI12719; Basic Yellow No. 57), Di(4-(dirnethylamino)phenyl)iminomethan-hydrochlorid (CI41000; Basic Yellow No. 2), Bis[4-(diethylamino)phenyl]phenylcarbeniumhydrogensulfat (1:1) (CI42040; Basic Green No. 1), Di(4-(dimethylamino)-phenyl)-phenylmethanol (CI42000; Basic Green No. 4), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 2-((4-(Acetylamino)phenyl)azo)-4-methylphenol (CI11855; Disperse Yellow No. 3), 6-Hydroxy-5-[(4-sulfophenyl)azo]-2-naphthalinsulfonsäuredinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (CI47005;D&C Yellow No. 10; Food Yellow No. 13; Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3Hxanthen-3-on (CI45350; Acid Yellow No. 73; D&C Yellow No. 8), 4-((4-Amino-3-sulfophenyl)azo)benzolsulfonsäure-dinatriumsalz (CI13015, Acid Yellow No. 9), 5-[(2,4-Dinitrophenyl)amino]-2-phenylaminobenzolsulfonsäure-natriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäuredinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäuredinatriumsalz (CI17200; Acid Red No. 33), 5-(Acetylamino)-4-hydroxy-3-[(2-methylphenyl)azol-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäuredinatriumsalz (CI27290; Acid Red No. 73), 2',4',5',7'-Tetrabrom-3',6'dihydroxyspiro[isobenzofuran-1(3H),9'-[9H]xanthen]-3-on-dinatriumsalz (CI45380; Acid Red No. 87), 2',4',5',7'-Tetrabrom-4,5,6,7-tetrachlor-3',6'dihydroxyspiro[isobenzofuran-1(3H),9'[9H)xanthen]-3-on-dinatriumsalz (CI45410; Acid Red No. 92), 3',6'-Dihydroxy-4',5'-diiodospiro[isobenzofuran-1(3H),9'(9H)-xanthen]-3-on-dinatriumsalz (CI45425; Acid Red No. 95), 2-Hydroxy-3-((2-hydroxynaphth-1-yl)azo)-5-nitrobenzolsulfonsäure-mononatriumsalz (CI15685; Acid Red No. 184), (2-Sulfophenyl)di[4-(ethyl((4-sulfophenyl)methyl)amino)phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinondinatriumsalz (CI 61570; Acid Green No. 25), Bis[4-(dimethylamino)-phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)carbeniuminneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatnumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)-amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalinsulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäuretetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäurenatriumsalz, Chrom-Komplex (Acid Red No. 195), 4-((5-((2-Hydroxyethyl)amino-1-methyl-1H-pyrazol-4-yl)imino)-4,5-dihydro-5-((2-hydroxyethyl)imino)-1-methyl-1H-pyrazol-monosulfat, 5-Hydroxy-1,4-naphthochinon (CI75500, Natural Brown No. 7), 2-Hydroxy-1,4-naphthochinon (CI75480, Natural Orange No. 6) und 1,2-Dihydra-2-(1,3-dihydro-3-oxo-2H-indol-2-yliden)-3H-indol-3-on (CI73000).

Die direktziehenden Farbstoffe können in der Farbträgermasse jeweils in einer Gesamtmenge von etwa 0,02 bis 20 Gewichtsprozent, vorzugsweise 0,2 bis 10 Gewichtsprozent, eingesetzt werden , wobei die Gesamtmenge an direktziehenden Farbstoffen in dem durch Vermischen der einzelnen Komponenten erhaltenen gebrauchsfertigen Färbemittel etwa 0,01 bis 10 Gewichtsprozent, vorzugsweise 0,1 bis 5 Gewichtsprozent, beträgt.

Die Farbträgermassen liegen vorzugsweise, ebenso wie das gebrauchsfertige Färbemittel, unabhängig voneinander jeweils in Form einer Lösung, insbesondere einer wäßrigen oder wäßrig-alkoholischen Lösung, einer Creme, eines Gels, einer Emulsion oder eines Aerosolschaumes vor.

Die Farbträgermassen können alle für derartige Zubereitungen üblichen und bekannten Stoffe enthalten, zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, femer Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die vorgenannten Stoffe werden in den für solche Zwecke üblichen Mengen verwendet, zum Beispiel die Netzmittel und Emulgatoren in Konzentrationen von etwa 0,5 bis 30 Gewichtsprozent (jeweils bezogen auf die einzelnen Farbträgermassen), die Verdicker in einer Menge von etwa 0,1 bis 25 Gewichtsprozent (jeweils bezogen auf die einzelnen Farbträgermassen) und die Pflegestoffe in einer Konzentration von etwa 0,1 bis 5,0 Gewichtsprozent (jeweils bezogen auf die einzelnen Farbträgermassen).

Der pH-Wert des gebrauchsfertigen Färbemittels stellt sich durch Vermischen der einzelnen Komponenten ein und beträgt etwa 3 bis 11, vorzugsweise 8 bis 11, insbesondere 9,5 bis 11. Eine besonders gute Lagerstabilität der Farbträgermassen ergibt sich, wenn die die Verbindungen der Formel (I) und/oder (II) und gegebenenfalls direktziehende Farbstoffe enthaltende Farbträgermasse (a) einen sauren pH-Wert von etwa 1,5 bis 6,8 aufweist und die die Aldehydverbindung sowie das Monoalkanolamin und gegebenenfalls direktziehende Farbstoffe enthaltende Farbträgermasse (b) einen alkalischen pH-Wert von etwa 9 bis 13 aufweist. Wird das erfindungsgemäße Färbemittel in Form eines 2-Komponenten-Mittels, bestehend aus einer Farbträgermasse (a), welche die Verbindungen der Formel (I) und/oder (II) sowie das Monoalkanolamin und gegebenenfalls direktziehende Farbstoffe enthält, und einer weiteren Farbträgermasse (b), welche die Aldehydverbindung und gegebenenfalls direktziehende Farbstoffe enthält, konfektioniert, so weist die Farbträgermasse (a) einen alkalischen pH-Wert von etwa 9 bis 13 auf, während die Farbträgermasse (b) einen sauren pH-Wert von etwa 1,5 bis 6,8 aufweist.

Zur Einstellung des für die Färbung geeigneten pH-Wertes können -falls erforderlich- zusätzlich zu dem Alkanolamin weitere alkalisierende Mittel wie zum Beispiel Alkylamine, Alkali- oder Erdalkalihydroxide oder Ammoniumhydroxid und Alkalicarbonate oder Ammoniumcarbonate, oder Säuren wie Milchsäure, Essigsäure, Weinsäure, Phosphorsäure, Salzsäure, Zitronensäure, Ascorbinsäure und Borsäure, verwendet werden.

Die einzelnen Komponenten werden unmittelbar vor der Anwendung vermischt und auf die Faser, beispielsweise Haare, aufgetragen. Je nach gewünschter Farbtiefe läßt man diese Mischung 5 bis 60 Minuten, vorzugsweise 15 bis 30 Minuten, bei einer Temperatur von 20 bis 50 Grad Celsius, insbesondere bei 30 bis 40 Grad Celsius einwirken. Anschließend wird die Faser mit Wasser gespült, gegebenenfalls mit einem Shampoo gewaschen und sodann getrocknet.

Die Verbindungen der Formel (I) und/oder (II) und die Aldehydverbindung werden in dem gebrauchsfertigen Färbemittel im allgemeinen in einem äquimolaren Verhältnis eingesetzt. Je nach gewünschtem Farbton können eine oder mehrere der Verbindungen der Formel (I) und/oder (II) mit einer oder mehreren Aldehydverbindungen vermischt werden, wodurch ein breites Spektrum an verschiedenen Nuancen erzeugt werden kann.

Die erfindungsgemäßen Färbemittel ergeben eine hervorragende Färbung von Keratinfasern, insbesondere von menschlichen Haaren, aber ebenso von Wolle oder Seide, in gelben bis blauen und grünen Farbtönen.

Überraschenderweise können diese Färbungen schnell und schonend durch Reduktionsmittel wieder vollständig entfärbt werden.

Ein weiter Gegenstand der vorliegenden Erfindung ist daher ein Mehrkomponenten-Kit zur Färbung und späteren Entfärbung von Fasern, wie zum Beispiel Wolle, Seide, Baumwolle oder Haaren und insbesondere menschlichen Haaren, welcher dadurch gekennzeichnet ist, daß er das erfindungsgemäße Färbemittel A und eine entfärbende Komponente B enthält, wobei die Komponente B als entfärbendes Agenz mindestens ein Sulfit und/oder Hydrogensulfit, beispelsweise ein Ammoniumhydrogensulfit, Ammoniumsulfit, Alkalisulfit oder Erdalkalisulfrt, insbesondere Natriumsulfit oder Ammoniumhydrogensulfit, und/oder Thioglykolsäure oder deren Salze enthält und einen pH-Wert von 4 bis 8 aufweist, wobei bei Verwendung von Thioglykolsäure oder deren Salze ein pH-Wert 7 bis 8 bevorzugt ist.

Die Gesamtmenge an Sulfit, Hydrogensulfit und Thioglykolsäure in der Komponente B beträgt etwa 0,1 bis 10 Gewichtsprozent, vorzugsweise 2 bis 5 Gewichtsprozent.

Das Mittel zur Entfärbung der dem Färbemittel A gefärbten Fasern (im folgenden "Entfärbemittel" genannt) kann als wäßrige oder wäßrigalkoholische Lösung, als Gel, Creme, Emulsion oder Schaum vorliegen, wobei das Entfärbemittel sowohl in Form eines Einkomponentenpräparats als auch in Form eines Mehrkomponentenpräparates konfektioniert sein kann. Das Entfärbemittel kann ebenfalls in Pulverform oder (zum Schutz vor Staubbildung) als Tablette - auch Brausetablette - oder Granulat konfektioniert sein. Hieraus wird dann vor der Anwendung mit kaltem oder warmem Wasser, gegebenenfalls unter Zusatz eines oder mehrerer der nachfolgend genannten Hilfsmittel, das Entfärbemittel hergestellt. Es ist jedoch auch möglich, daß diese Hilfsmittel (sofern sie in fester Form vorliegen) bereits in dem Entfärbepulver oder Entfärbegranulat beziehungsweise der Tablette enthalten sind. Durch Benetzung des Pulvers durch Öle oder Wachse kann zusätzlich die Staubbildung vermindert werden.

Das Entfärbemittel kann zusätzliche Hilfsmittel, wie zum Beispiel Lösungsmittel wie Wasser, niedere aliphatische Alkohole, beispielsweise Ethanol, n-Propanol und Isopropanol, Glykolether oder Glykole wie Glycerin und insbesondere 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, femer Verdicker wie höhere Fettalkohole, Stärke oder Cellulosederivate, Parfüme, Haarvorbehandlungsmittel, Konditionierer, Haarquellmittel, Konservierungsstoffe, Vaseline, Paraffinöl und Fettsäuren sowie außerdem Pflegestoffe wie kationische Harze, Lanolinderivate, Cholesterin, Pantothensäure und Betain, enthalten.

Der pH-Wert des Entfärbemittels beträgt etwa 4 bis 8, insbesondere etwa 3 bis 7,5. Erforderlichenfalls kann der gewünschte pH-Wert durch Zugabe von geeigneten Säuren, beispielsweise a-Hydroxycarbonsäuren wie Milchsäure, Weinsäure, Zitronensäure oder Äpfelsäure, Phosphorsäure, Essigsäure, Glycolsäure Salicylsäure, Glutathion oder Gluconsäurelacton, oder aber alkalisierenden Mitteln wie Alkanolaminen, Alkylaminen, Alkalihydroxiden, Ammoniumhydroxid, Alkalicarbonaten, Ammoniumcarbonaten oder Alkaliphosphaten, eingestellt werden.

Die Einwirkungszeit des Entfärbemittels beträgt je nach zu entfärbender Färbung und Temperatur (etwa 20 bis 50 Grad Celsius) 5 bis 60 Minuten, insbesondere 15 bis 30 Minuten, wobei durch Wärmezufuhr der Entfärbeprozeß beschleunigt werden kann. Nach Beendigung der Einwirkungszeit des Entfärbemittels wird das Haar mit Wasser gespült, gegebenenfalls mit einem Shampoo gewaschen.

Die nachfolgenden Beispiele sollen den Gegenstand näher erläutern, ohne ihn auf diese Beispiele zu beschränken.

### Beispiele

### Beispiele 1 bis 10: Haarfärbemittel

### Verbindung der Formel (I) oder (II) enthaltende Komponente (a)

| | |
|---|---|
| Verbindung der Formel **(I)** oder **(II)** | Mengenangaben gemäß Tabelle 1 |
| 6-O-Palmitoyl-L-ascorbinsäure | 0,30 g |
| Cetylstearylalkohol | 12,00 g |
| Laurylethersulfat, 28-prozentige wässrige Lösung | 10,00 g |
| Ethanol | 23,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird die Verbindung (I) oder (II), versetzt mit dem restlichen Wasser und dem Alkohol und der 6-O-Palmitoyl-L-ascorbinsäure, zugegeben. Der pH-Wert der Creme wird mit Monoethanolamin auf 12,0 eingestellt.

### Aldehydhaltige Komponente (b)

| | |
|---|---|
| Aldehydverbindung | Mengenangaben gemäß Tabelle 1 |
| direktziehender Farbstoff | Mengenangaben gemäß Tabelle 1 |
| Cetylstearylalkohol | 12,00 g |
| Laurylethersulfat, 28-prozentige wässrige Lösung | 10,00 g |
| 6-O-Palmitoyl-L-ascorbinsäure | 0,30 g |
| Ethanol | 23,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird die Aldehydverbindung, versetzt mit dem restlichen Wasser und dem Alkohol und der 6-O-Palmitoyl-L-ascorbinsäure, zugegeben. Der pH-Wert der Creme wird mit 10 %iger wäßriger Milchsäure auf 4,0 eingestellt.

5 g der Komponente (a) werden mit 5 g der Komponente (b) vermischt. Der pH-Wert der Fertigmischung wird gegebenenfalls mit Monoethanolamin auf pH 10,5 eingestellt. Das erhaltene gebrauchsfertige Haarfärbemittel wird auf eine Haarsträhne aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die erhaltenen Färbungen sind in der nachfolgenden Tabelle 1 zusammengefaßt.

### Beispiele 11 bis 13: Haarfärbemittel

### Verbindung der Formel (I) enthaltende Komponente (a)

| | |
|---|---|
| Verbindung der Formel **(I)** | Mengenangaben gemäß Tabelle 2 |
| Cetylstearylalkohol | 12,0 g |
| Laurylethersulfat, 28-prozentige wässrige Lösung | 10,0 g |
| Ethanol | 25,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird die Verbindung der Formel (I), versetzt mit dem restlichen Wasser und dem Alkohol, zugegeben.

### Aldehydhaltige Komponente (b)

| | |
|---|---|
| Aldehydverbindung | Mengenangaben gemäß Tabelle 2 |
| Cetylstearylalkohol | 12,0 g |
| Laurylethersulfat, 28-prozentige wässrige Lösung | 10,0 g |
| Ethanol | 25,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird der Aldehyd, versetzt mit dem restlichen Wasser und dem Alkohol, zugegeben. Der pH-Wert der Creme wird mit Monoethanolamin auf 10,2 eingestellt.

5 g der Komponente (a) werden mit 5 g der Komponente (b) vermischt. Der pH-Wert der Fertigmischung wird gegebenenfalls mit Monoethanolamin auf pH 10,0 eingestellt. Das erhaltene gebrauchsfertige Haarfärbemittel wird auf eine Haarsträhne aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die erhaltenen Färbungen sind in der nachfolgenden Tabelle 2 zusammengefaßt.

### Beispiele 14 bis 16: Haarfärbemittel

### Verbindung der Formel (II) enthaltende Komponente (a)

| | |
|---|---|
| Verbindung der Formel **(II)** | Mengenangaben gemäß Tabelle 3 |
| Cetylstearylalkohol | 12,00 g |
| Laurylethersulfat, 28 %ige wässrige Lösung | 10,00 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird die Verbindung (11), mit dem restlichen Wasser versetzt, zugegeben.

### Aldehydhaltige Komponente (b)

| | |
|---|---|
| Aldehydverbindung | Mengenangaben gemäß Tabelle 3 |
| Cetylstearylalkohol | 12,0 g |
| Laurylethersulfat, 28-prozentige wässrige Lösung | 10,0 g |
| Wasser, vollentsalzt | ad 100,0 g |

Der Cetylstearylalkohol wird bei 80 °C geschmolzen. Das Laurylethersulfat wird mit 95 % des Wassers auf 80 °C erhitzt und zum geschmolzenen Cetylstearylalkohol gegeben und gerührt bis sich eine Creme ergibt. Bei Raumtemperatur wird der Aldehyd, versetzt mit dem restlichen Wasser, zugegeben. Der pH-Wert der Creme wird mit Monoethanolamin auf 10,2 eingestellt.

5 g der Komponente (a) werden mit 5 g der Komponente (b) vermischt. Der pH-Wert der Fertigmischung wird gegebenenfalls mit Monoethanolamin auf pH 10,0 eingestellt. Das erhaltene gebrauchsfertige Haarfärbemittel wird auf eine Haarsträhne aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die erhaltenen Färbungen sind in der nachfolgenden Tabelle 3 zusammengefaßt.

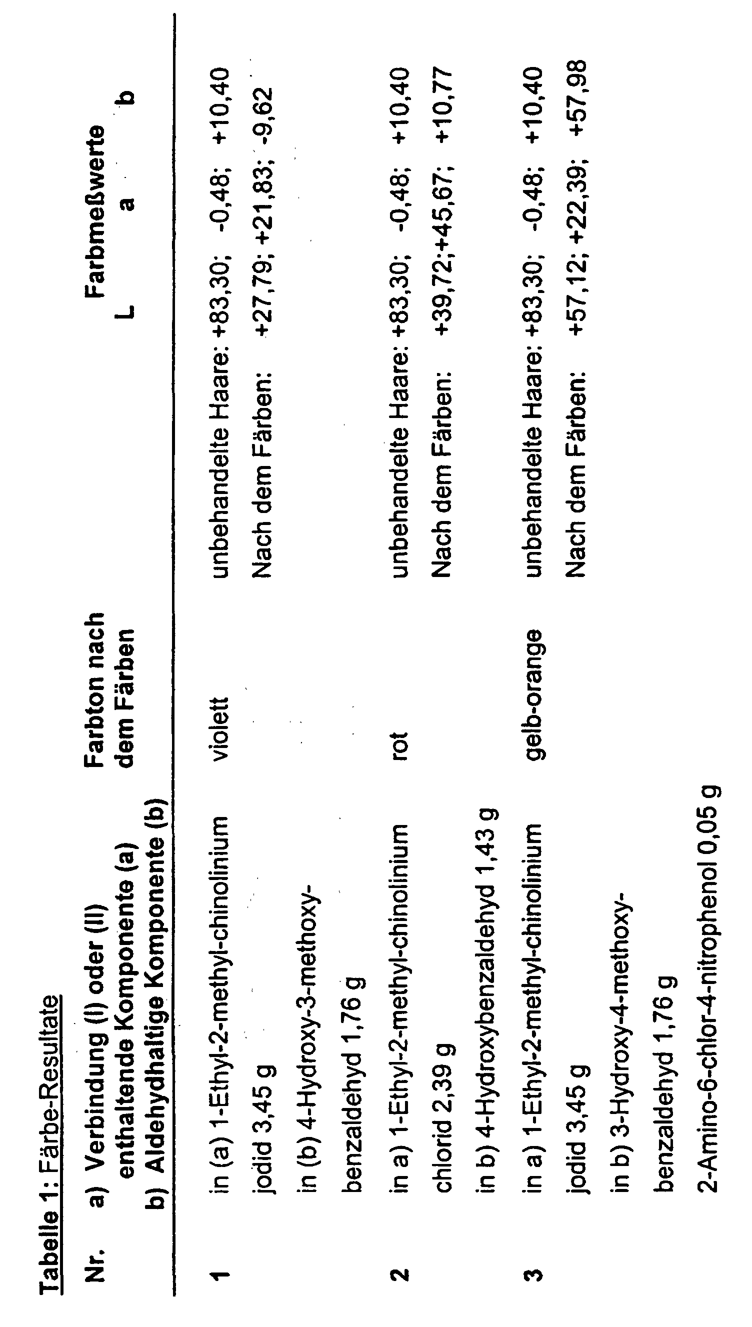

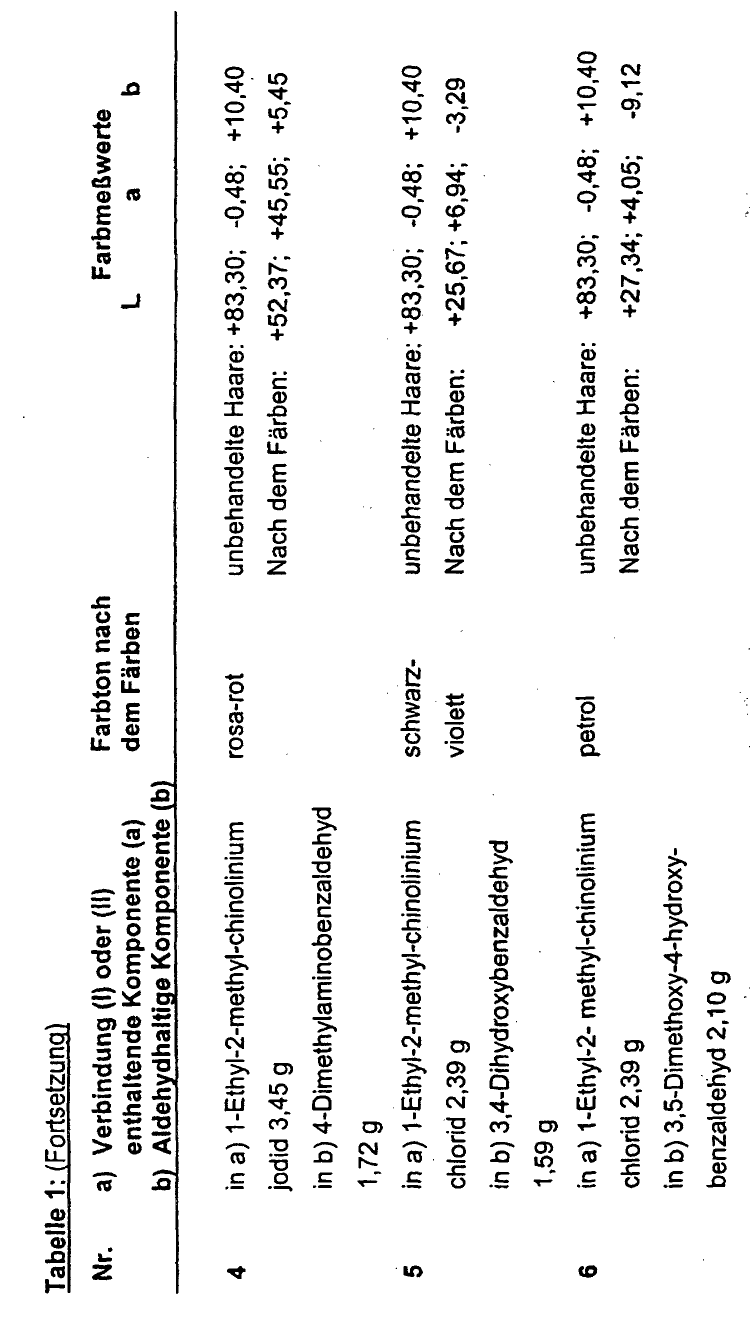

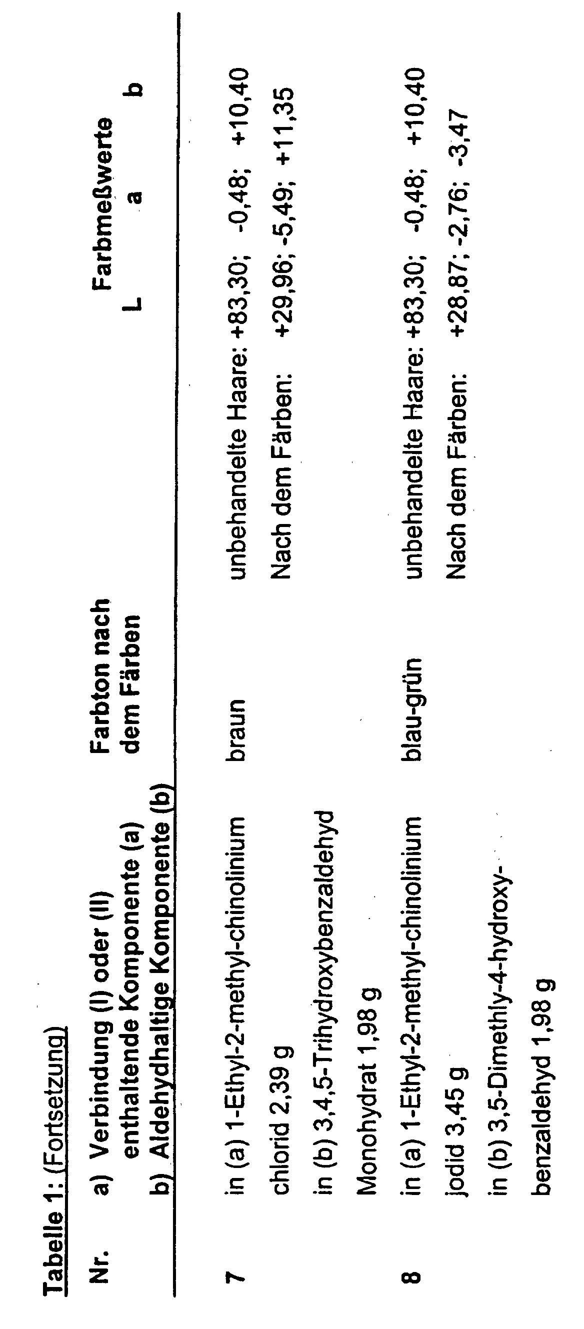

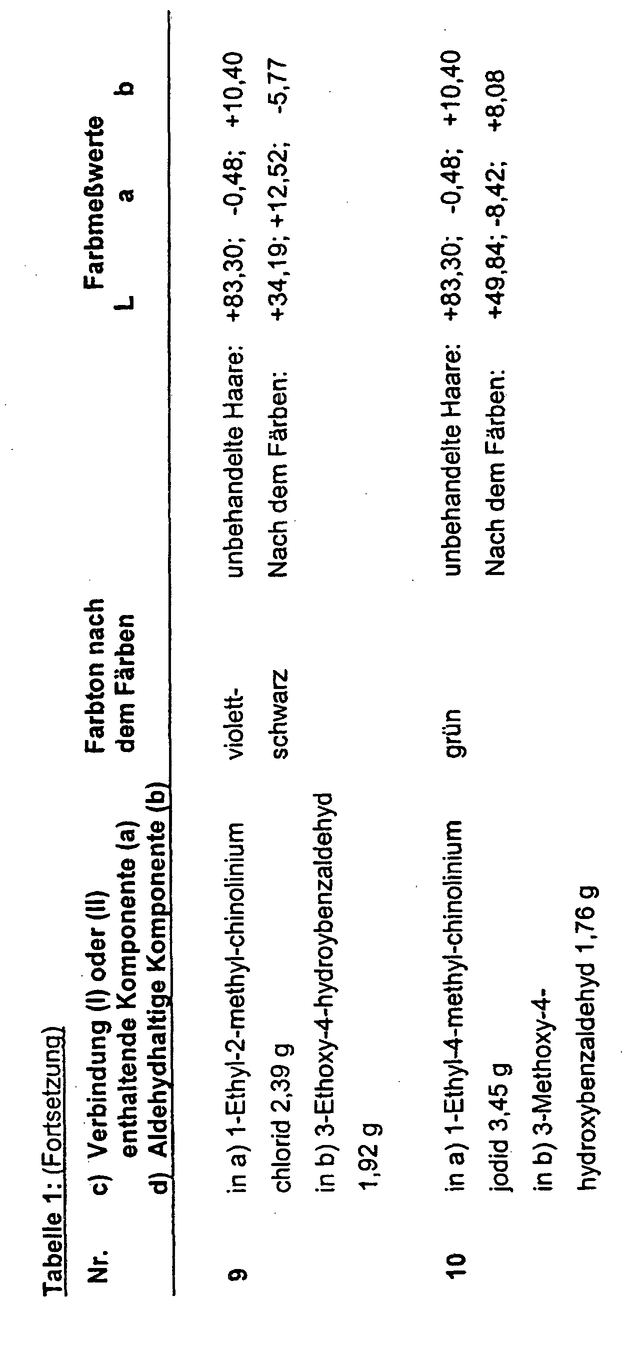

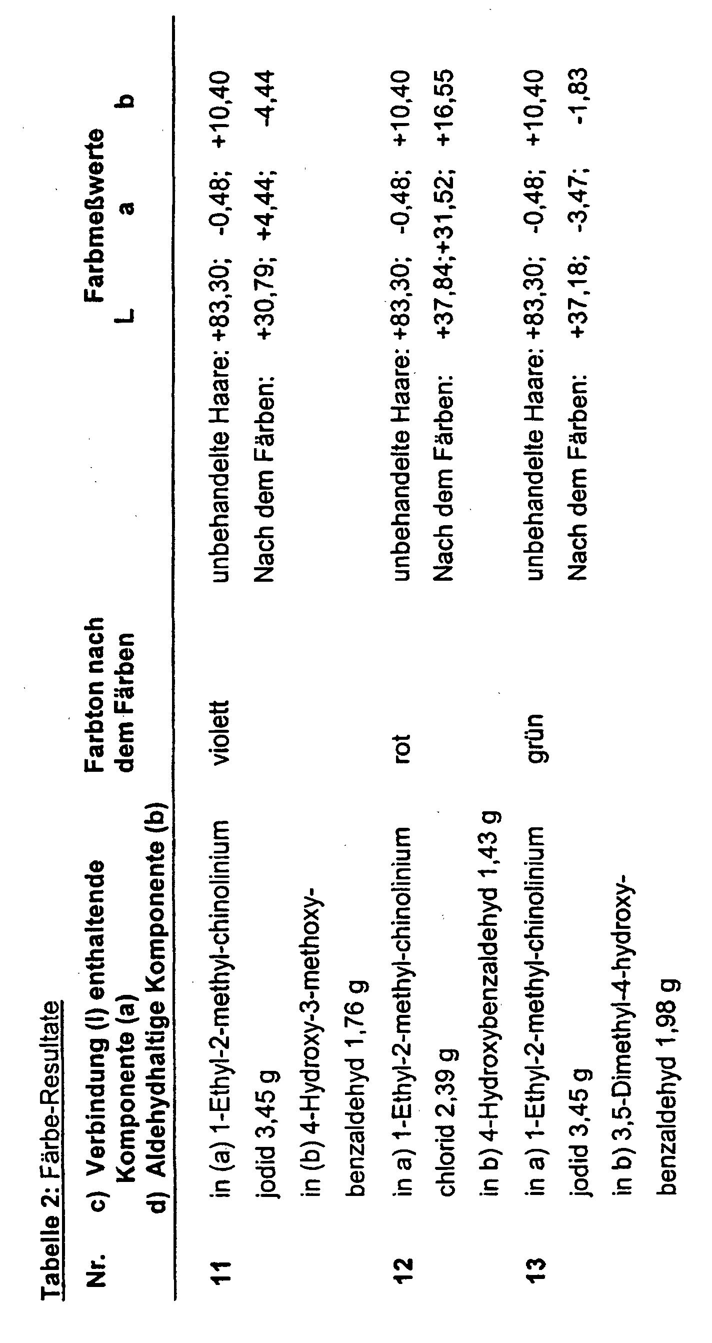

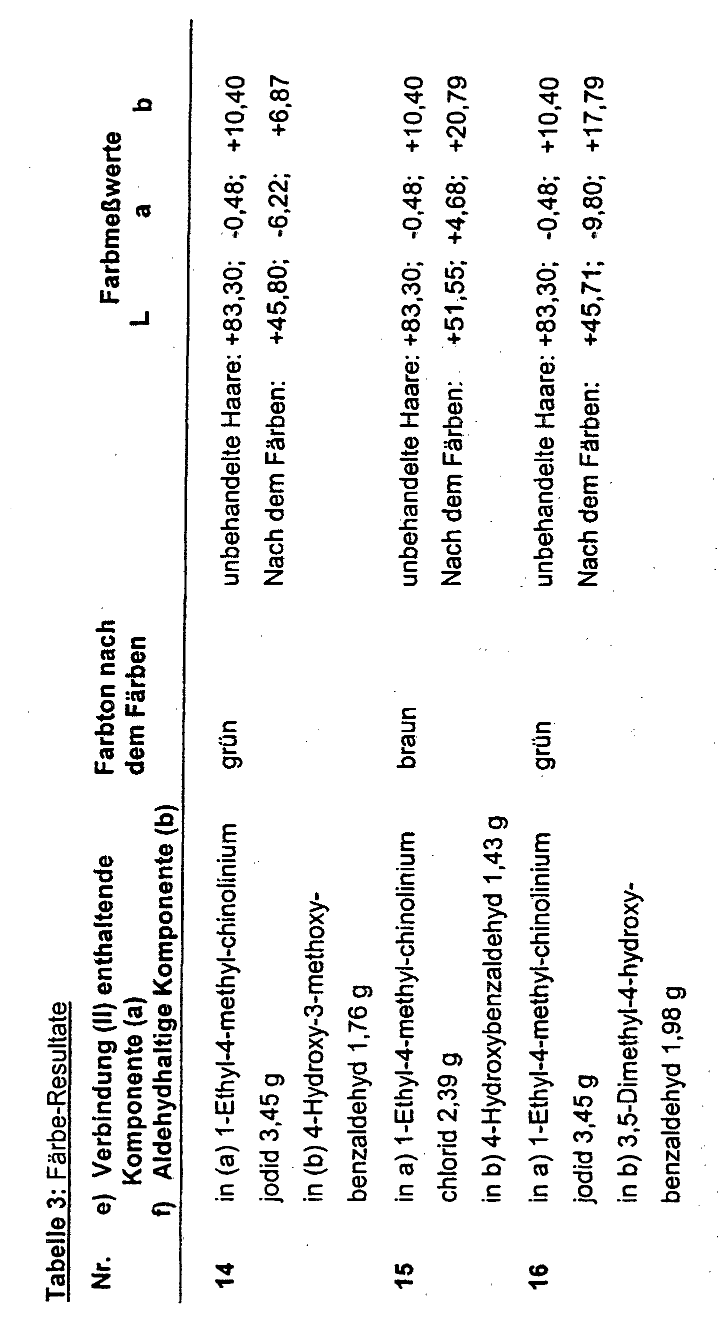

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

## Patentansprüche

1. Mittel zur Färbung von Fasern, **dadurch gekennzeichnet, daß** es ein Alkanolamin, mindestens eine aromatische Aldehydverbindung, und mindestens eine Verbindung der Formel (I) oder (II), 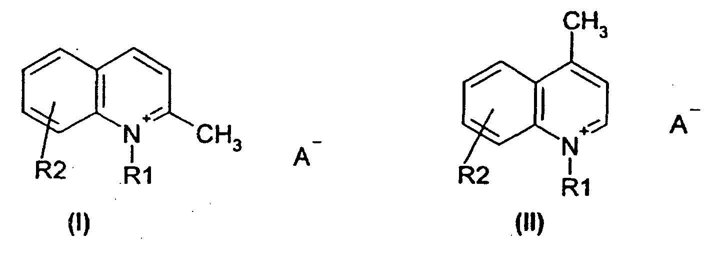 in der R1 für eine C1-C3-Alkylgruppe, C1-C3-Alkoxyalkylgruppe oder C1-C3-Hydroxyalkylgruppe steht und R2 gleich Wasserstoff, einer Hydroxygruppe, einer Methoxygruppe, einem Halogenatom , einer Aminogruppe oder einer Dimethylaminogruppe ist und A⁻ ein Anion, beispielsweise Chlorid, Bromid, Sulfat, Hydrogensulfat, Monomethylsulfat, Acetat, Lactat oder Jodid, darstellt, enthält.

2. Mittel nach Anspruch 1, das durch Vermischen zweier Komponenten erhalten wird, **dadurch gekennzeichnet, daß** die eine Komponente (Komponente (b)) mindestens eine aromatische Aldehydverbindung enthält und die andere Komponente (Komponente (a)) mindestens eine Verbindung der Formel (I) oder (II) sowie ein Alkanolamin enthält.

3. Mittel nach Anspruch 1, das durch Vermischen zweier Komponenten erhalten wird, **dadurch gekennzeichnet, daß** die eine Komponente (Komponente (b)) mindestens eine aromatische Aldehydverbindung sowie ein Alkanolamin enthält und die andere Komponente (Komponente (a)) mindestens eine Verbindung der Formel (I) oder (II) enthält.

4. Mittel nach Anspruch1, das durch Vermischen zweier Komponenten erhalten wird, **dadurch gekennzeichnet, daß** die eine Komponente mindestens eine aromatische Aldehydverbindung und mindestens eine Verbindung der Formel (I) oder (II) enthält und die andere Komponente ein Alkanolamin enthält.

5. Mittel nach Anspruch1, das durch Vermischen dreier Komponenten erhalten wird, **dadurch gekennzeichnet, daß** die eine Komponente (Komponente (b)) mindestens eine aromatische Aldehydverbindung enthält, die andere Komponente (Komponente (a)) mindestens eine Verbindung der Formel (I) oder (II) enthält sowie die dritte Komponente ein Alkanolamin enthält.

6. Mittel nach einem der Ansprüche 1 bis 5, **dadurch gekennzeichnet, daß** die Verbindung der Formel (I) oder (II) ausgewählt ist aus 1-Ethyl-2-methyl-chinoliniumjodid, 1-Ethyl-2-methyl-chinoliniumchlorid, 1-Ethyl-4-methyl-chinoliniumjodid und 1-Ethyl-4-methyl-chinoliniumchlorid.

7. Mittel nach einem der Ansprüche 1 bis 6, **dadurch gekennzeichnet, daß** die aromatische Aldehydverbindung ausgewählt ist aus Vanillin, Isovanillin, 3,4-Dihydroxy-benzaldehyd, 4-Hydroxybenzaldehyd, 3,5-Dimethoxy-4-hydroxy-benzaldehyd, 4-Dimethylaminobenzaldehyd, 4- Dimethylamino-zimtaldehyd, 4-Hydroxy-2-methoxy-benzaldehyd, 3,5-Dimethyl-4-hydroxybenzaldehyd, 4-Dimethylamino-2-methoxybenzaldehyd, 2-Hydroxybenzaldehyd, 4-Hydroxy-1-naphthaldehyd, 4-Methoxy-1-naphthaldehyd, 4-Dimethylamino-1-naphthaldehyd, 4'-Hydroxy-biphenyl-1-carbaldehyd, 2-Hydroxy-3-methoxybenzaldehyd, 2,4-Dihydroxy-benzaldehyd, 3,4-Dihydroxybenzaldehyd, 2,5-Dihydroxybenzaldehyd, 2,3,4-Trihydroxybenzaldehyd, 3,4,5-Trihydroxybenzaldehyd, 2,4,6-Trihydroxybenzaldehyd, 2,4-Dimethoxybenzaldehyd, 2,3-Dimethoxybenzaldehyd, 2,5-Dimethoxybenzaldehyd, 3,5-Dimethoxy-benzaldehyd, 3,4-Dimethoxy-benzaldehyd, Benzol-1,4-dicarbaldehyd, 4-Ethoxy-benzaldehyd, 2-Methyl-1,4-naphthochinon, 4-Carboxybenzatdehyd, 4-Hydroxy-3-methoxyzimtaldehyd, 3,5-Dimethoxy-4-hydroxy-zimtaldehyd, 3-Methoxy-4-(1-pyrrolidinyl)-benzaldehyd, 4-Diethylamino-3-methoxybenzaldehyd, 1,2-Phthaldialdehyd, 4-Dibutylamino-benzaldehyd, 4-Diethylamino-2-hydroxybenzaldehyd, 3,4-Dimethoxy-5-hydroxybenzaldehyd, 5-(4-(Diethylamino)phenyl)-2,4-pentadienal, 2-Methoxy-1-naphthaldehyd, 3-Ethoxy-4-hydroxybenzaldehyd, 2-Nitrobenzaldehyd, 3-Nitrobenzaldehyd und 4-Nitrobenzaldehyd.

8. Mittel nach einem der Ansprüche 1 bis 7, **dadurch gekennzeichnet, daß** die Verbindung der Formel (I) oder (II) und die Aldehydverbindung in dem gebrauchsfertigen Mittel jeweils in einer Menge von 0,01 bis 10 Gewichtsprozent enthalten sind.

9. Mittel nach einem der Ansprüche 1 bis 8, **dadurch gekennzeichnet, daß** das Alkanolamin ein Monoethanolamin ist.

10. Mittel nach einem der Ansprüche 1 bis 9, **dadurch gekennzeichnet, daß** es zusätzlich direktziehende Farbstoffe enthält.

11. Mittel nach Anspruch 3, **dadurch gekennzeichnet, daß** die Komponente (a) einen pH-Wert von 1,5 bis 6,8 und die Komponente (b) einen pH-Wert von 9 bis 13 aufweist.

12. Mittel nach einem der Ansprüche 1 bis 11, **dadurch gekennzeichnet, daß** die durch Vermischen der einzelnen Komponenten erhaltene gebrauchsfertige Zubereitung einen pH-Wert von 3 bis 11 aufweist.

13. Verfahren zum Färben von Haaren, **dadurch gekennzeichnet, daß** man ein Mittel nach einem der Ansprüche 1 bis 12 auf das Haar aufträgt, für eine Dauer von 5 bis 60 Minuten bei einer Temperatur von 20 bis 50 °C auf die Fasern einwirken läßt, sodann das Haar mit Wasser spült und anschließend trocknet.

14. Mehrkomponenten-Kit zur Färbung und späteren Entfärbung von Fasern, **dadurch gekennzeichnet, daß** er ein Färbemittel A gemäß einem der Ansprüche 1 bis 12 und eine entfärbende Komponente B enthält, wobei die Komponente B als entfärbendes Agenz mindestens ein Sulfit und/oder Hydrogensulfit und/oder Thioglykolsäure oder deren Salze enthält und einen pH-Wert von 4 bis 8 aufweist.

## Claims

1. Agent for colouring fibres, **characterized in that** it comprises an alkanolamine, at least one aromatic aldehyde compound, and at least one compound of the formula (I) or (II), 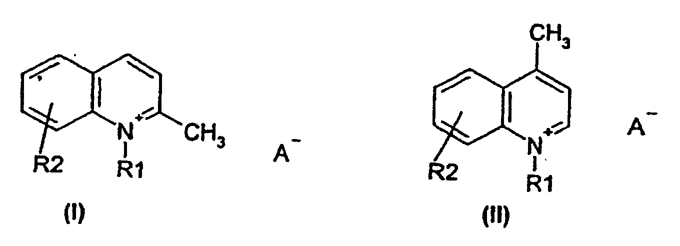 in which R1 is a C1-C3-alkyl group, C1-3-alkoxyalkyl group or C1-C3-hydroxyalkyl group, and R2 is hydrogen, a hydroxyl group, a methoxy group, a halogen atom, an amino group or a dimethylamino group, and A⁻ is an anion, for example chloride, bromide, sulphate, hydrogensulphate, monomethylsulphate, acetate, lactate or iodide.

2. Agent according to Claim 1, which is obtained by mixing two components, **characterized in that** the one component (component (b)) comprises at least one aromatic aldehyde compound, and the other component (component (a)) comprises at least one compound of the formula (I) or (II), and an alkanolamine.

3. Agent according to Claim 1, which is obtained by mixing two components, **characterized in that** the one component (component (b)) comprises at least one aromatic aldehyde compound and an alkanolamine, and the other component (component (a)) comprises at least one compound of the formula (I) or (II).

4. Agent according to Claim 1, which is obtained by mixing two components, **characterized in that** the one component comprises at least one aromatic aldehyde compound and at least one compound of the formula (I) or (II), and the other component comprises an alkanolamine.

5. Agent according to Claim 1, which is obtained by mixing three components, **characterized in that** the one component (component (b)) comprises at least one aromatic aldehyde compound, the other component (component a)) comprises at least one compound of the formula (I) or (II), and the third component comprises an alkanolamine.

6. Agent according to one of Claims 1 to 5, **characterized in that** the compound of the formula (I) or (II) is chosen from 1-ethyl-2-methylquinolinium iodide, 1-ethyl-2-methylquinolinium chloride, 1-ethyl-4-methylquinolinium iodide and 1-ethyl-4-methylquinolinium chloride.

7. Agent according to one of Claims 1 to 6, **characterized in that** the aromatic aldehyde compound is chosen from vanillin, isovanillin, 3,4-dihydroxybenzaldehyde, 4-hydroxybenzaldehyde, 3,5-dimethoxy-4-hydroxybenzaldehyde, 4-dimethylaminobenzaldehyde, 4-dimethylaminocinnamaldehyde, 4-hydroxy-2-methoxybenzaldehyde, 3,5-dimethyl-4-hydroxybenzaldehyde, 4-dimethylamino-2-methoxybenzaldehyde, 2-hydroxybenzaldehyde, 4-hydroxy-1-naphthaldehyde, 4-methoxy-1-naphthaldehyde, 4-dimethylamino-1-naphthaldehyde, 4'-hydroxybiphenyl-1-carbaldehyde, 2-hydroxy-3-methoxybenzaldehyde, 2,4-dihydroxybenzaldehyde, 3,4-dihydroxybenzaldehyde, 2,5-dihydroxybenzaldehyde, 2,3,4-trihydroxybenzaldehyde, 3,4,5-trihydroxybenzaldehyde, 2,4,6-trihydroxybenzaldehyde, 2,4-dimethoxybenzaldehyde, 2,3-dimethoxybenzaldehyde, 2,5-dimethoxybenzaldehyde, 3.5-dimethoxybenzaldehyde, 3,4-dimethoxybenzaldehyde, benzene-1,4-dicarbaldehyde, 4-ethoxybenzaldehyde, 2-methyl-1,4-naphthoquinone, 4-carboxybenzaldehyde, 4-hydroxy-3-methoxycinnamaldehyde, 3,5-dimethoxy-4-hydroxycinnamaldehyde, 3-methoxy-4-(1-pyrrolidinyl)benzaldehyde, 4-diethylamino-3-methoxybenzaldehyde, 1,2-phthaldialdehyde, 4-dibutylaminobenzaldehyde, 4-diethylamino-2-hydroxybenzaldehyde, 3,4-dimethoxy-5-hydroxybenzaldehyde, 5-(4-(diethylamino)phenyl-2,4-pentadienal, 2-methoxy-1-naphthaldehyde, 3-ethoxy-4-hydroxybenzaldehyde, 2-nitrobenzaldehyde, 3-nitrobenzaldehyde and 4-nitrobenzaldehyde.

8. Agent according to one of Claims 1 to 7, **characterized in that** the compound of the formula (I) or (II) and the aldehyde compound in the ready-to-use agent are in each case present in an amount of from 0.01 to 10% by weight.

9. Agent according to one of Claims 1 to 8, **characterized in that** the alkanolamine is a monoethanolamine.

10. Agent according to one of Claims 1 to 9, **characterized in that** it additionally comprises direct dyes.

11. Agent according to Claim 3, **characterized in that** the component (a) has a pH of from 1.5 to 6.8 and the component (b) has a pH of from 9 to 13.

12. Agent according to one of Claims 1 to 11, **characterized in that** the ready-to-use preparation obtained by mixing the individual components has a pH of from 3 to 11.

13. Method of colouring hair, **characterized in that** an agent as claimed in one of Claims 1 to 12 is applied to the hair, left to act on the fibres for a period of from 5 to 60 minutes at a temperature of from 20 to 50°C, then the hair is rinsed with water and subsequently dried.

14. Multicomponent kit for colouring and subsequent decolouring of fibres, **characterized in that** it comprises a colorant A according to one of Claims 1 to 12 and a decolouring component B, where the component B comprises, as decolouring agent, at least one sulphite and/or hydrogen sulphite and/or thioglycolic acid or salts thereof and has a pH of from 4 to 8.

## Revendications

1. Composition pour la teinture de fibres, **caractérisée en ce qu'**elle contient une alcanolamine, au moins un composé aldéhydique aromatique et au moins un composé de formule (I) ou (II) 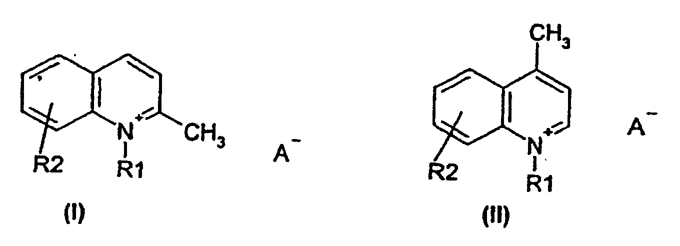 dans laquelle R1 représente un groupe alkyle en C₁-C₃, un groupe alcoxyalkyle en C₁-C₃ ou un groupe hydroxyalkyle en C₁-C₃ et R2 représente un atome d'hydrogène, un groupe hydroxy, un groupe méthoxy, un atome d'halogène, un groupe amino ou un groupe diméthylamino et A⁻ représente un anion, par exemple chlorure, bromure, sulfate, hydrogénosulfate, monométhylsulfate, acétate, lactate ou iodure.

2. Composition selon la revendication 1, qui est obtenue par mélange de deux composants, **caractérisée en ce qu'**un composant [composant (b)] contient au moins un composé aldéhydique aromatique et l'autre composant [composant (a)] contient au moins un composé de formule (I) ou (II) ainsi qu'une alcanolamine.

3. Composition selon la revendication 1, qui est obtenue par mélange de deux composants, **caractérisée en ce qu'**un composant [composant (b)] contient au moins un composé aldéhydique aromatique ainsi qu'une alcanolamine et l'autre composant [composant (a)] contient au moins un composé de formule (I) ou (II).

4. Composition selon la revendication 1, qui est obtenue par mélange de deux composants, **caractérisée en ce qu'**un composant contient au moins un composé aldéhydique aromatique et au moins un composé de formule (I) ou (II) et l'autre composant contient une alcanolamine.

5. Composition selon la revendication 1, qui est obtenue par mélange de trois composants, **caractérisée en ce qu'**un composant [composant (b)] contient au moins un composé aldéhydique aromatique, l'autre composant [composant (a)] contient au moins un composé de formule (I) ou (II) et le troisième composant contient une alcanolamine.

6. Composition selon l'une quelconque des revendications 1 à 5, **caractérisée en ce que** le composé de formule (I) ou (II) est choisi parmi l'iodure de 1-éthyl-2-méthylquinolinium, le chlorure de 1-éthyl-2-méthylquinolinium, l'iodure de 1-éthyl-4-méthylquinolinium et le chlorure de 1-éthyl-4-méthylquinolinium.

7. Composition selon l'une quelconque des revendications 1 à 6, **caractérisée en ce que** le composé aldéhydique aromatique est choisi parmi la vaniline, l'isovaniline, le 3,4-dihydroxybenzaldéhyde, le 4-hydroxybenzaldéhyde, le 3,5-diméthoxy-4-hydroxybenzaldéhyde, le 4-diméthylaminobenzaldéhyde, le 4-diméthylaminocinnamaldéhyde, le 4-hydroxy-2-méthoxybenzaldéhyde, le 3,5-diméthyl-4-hydroxybenzaldéhyde, le 4-diméthylamino-2-méthoxybenzaldéhyde, le 2-hydroxybenzaldéhyde, le 4-hydroxy-1-naphtaldéhyde, le 4-méthoxy-1-naphtaldéhyde, le 4-diméthylamino-1-naphtaldéhyde, le 4'-hydroxybiphényl-1-carbaldéhyde, le 2-hydroxy-3-méthoxybenzaldéhyde, le 2,4-dihydroxybenzaldéhyde, le 3,4-dihydroxybenzaldéhyde, le 2,5-dihydroxybenzaldéhyde, le 2,3,4-trihydroxybenzaldéhyde, le 3,4,5-trihydroxybenzaldéhyde, le 2,4,6-trihydroxybenzaldéhyde, le 2,4-diméthoxybenzaldéhyde, le 2,3-diméthoxybenzaldéhyde, le 2,5-diméthoxybenzaldéhyde, le 3,5-diméthoxybenzaldéhyde, le 3,4-diméthoxybenzaldéhyde, le benzène-1,4-dicarbaldéhyde, le 4-éthoxybenzaldéhyde, la 2-méthyl-1,4-naphtoquinone, le 4-carboxybenzaldéhyde, le 4-hydroxy-3-méthoxycinnamaldéhyde, le 3,5-diméthoxy-4-hydroxycinnamaldéhyde, le 3-méthoxy-4-(1-pyrrolidinyl)-benzaldéhyde, le 4-diéthylamino-3-méthoxybenzaldéhyde, le 1,2-phtaldialdéhyde, le 4-dibutylaminobenzaldéhyde, le 4-diéthylamino-2-hydroxybenzaldéhyde, le 3,4-diméthoxy-5-hydroxybenzaldéhyde, le 5-(4-(diéthylamino)phényl)-2,4-pentadiénal, le 2-méthoxy-1-naphtaldéhyde, le 3-éthoxy-4-hydroxybenzaldéhyde, le 2-nitrobenzaldéhyde, le 3-nitrobenzaldéhyde et le 4-nitrobenzaldéhyde.

8. Composition selon l'une quelconque des revendications 1 à 7, **caractérisée en ce que** le composé de formule (I) ou (II) et le composé aldéhydique sont contenus chacun dans la composition prête à l'emploi en une quantité de 0,01 à 10 % en poids.

9. Composition selon l'une quelconque des revendications 1 à 8, **caractérisée en ce que** l'alcanolamine est une monoéthanolamine.

10. Composition selon l'une quelconque des revendications 1 à 9, **caractérisée en ce qu'**elle contient en outre des colorants directs.

11. Composition selon la revendication 3, **caractérisée en ce que** le composant (a) a un pH de 1,5 à 6,8 et le composant (b) a un pH de 9 à 13.

12. Composition selon l'une quelconque des revendications 1 à 11, **caractérisée en ce que** la préparation prête à l'emploi, obtenue par mélange des composants individuels, a un pH de 3 à 11.

13. Procédé pour la teinture des cheveux, **caractérisé en ce qu'**on applique sur les cheveux une composition selon l'une quelconque des revendication 1 à 12, on laisse agir sur les fibres pendant une durée de 5 à 60 minutes, à une température de 20 à 50°C, puis on rince les cheveux à l'eau et ensuite on les sèche.

14. Nécessaire à plusieurs composants pour la coloration et la décoloration ultérieure de fibres, **caractérisé en ce qu'**il contient une composition de teinture A selon l'une quelconque des revendications 1 à 12 et un composant décolorant B, le composant B comportant en tant qu'agent décolorant au moins un sulfite et/ou hydrogénosulfite et/ou de l'acide thioglycolique ou ses sels et présente un pH de 4 à 8.
